# EUROPEAN PATENT APPLICATION

(11) **EP 2 078 700 A1**
(43) Date of publication of application: **15.07.2009**
(21) Application number: 08171864.5
(22) Date of filing: 16.12.2008
(51) Int. Cl.: C02F 1/00, C02F 1/46, C02F 1/461, C02F 1/467

(54) **Electrolyzed water generating and spraying device**

(30) Priority: 25.12.2007 JP 2007331333
(71) Applicant: MIKUNI CORPORATION, Chiyoda-ku, Tokyo (JP)
(72) Inventor: Kasuya, Shoji, Kanagawa (JP); Pang, Xilong, Kanagawa (JP); Hashimoto, Hiroshi, Kanagawa (JP)
(74) Representative: Betten & Resch

(57) **Abstract**

The invention provides an electrolyzed water generating and spraying device that allows generating electrolyzed water in a short time, and allows spraying non-deteriorated electrolyzed water immediately after being generated. An electrolyzed water generating and spraying device has a container, and housed therein, an electrolytic cell for generating electrolyzed water through electrolysis of an electrolytic solution by an electrode, a manual spray pump (spraying mechanism) for manually spraying the electrolyzed water generated in the electrolytic cell, and a power supply (battery) for energizing the electrode. A tank for storing the electrolytic solution and the electrolytic cell are provided independently in the container, such that the electrolytic cell is disposed below the tank, and the tank and the electrolytic cell communicated with each other via a communicating passage. Part of at least the electrolytic cell in the container is transparent. Also, the volume of the electrolytic cell is set to be smaller than the volume of the tank.

## Description

### BACKGROUND OF THE INVENTION

### 1. Field of the Invention

The present invention relates to an electrolyzed water generating and spraying device for spraying and applying electrolyzed water, obtained by electrolysis of an electrolytic solution, onto human skin or the like.

### 2. Description of the Related Art

Electrolyzed water obtained through electrolysis of an electrolytic solution is sprayed and applied onto human skin or the like, for instance to clean, disinfect and sterilize the skin thereby. Electrolyzed water generating devices, for generating electrolyzed water, and spraying devices, for spraying the electrolyzed water generated by the electrolyzed water generating device, have conventionally been constructed independently. Accordingly, the electrolyzed water generated in the electrolyzed water generating device must be transferred to the spraying device, to be sprayed therefrom. This operation is problematic on account of the hassle involved.

Such being the case, Japanese Patent No. 2796075 proposes an electrolyzed water generating and spraying device in which a container (tank) holding an electrolytic solution and doubling as an electrolytic cell is integrated as a single unit with a spraying mechanism for spraying electrolyzed water.

The electrolyzed water generating and spraying device proposed in Japanese Patent No. 2796075, however, relies on a batch-wise operation whereby the entire electrolytic solution in the container is electrolyzed. Thus, electrolyzed water cannot be sprayed until electrolysis of the electrolytic solution in the container is completely over, which is problematic in terms of the time that elapses until spraying.

The electrolyzed water generating and spraying device is also problematic in that the electrolyzed water in the container must be stirred, and also in terms of electrolyzed water deterioration, since the electrolyzed water remains in the container after being generated.

The device is problematic, moreover, in that re-electrolysis efficiency is poor, since the entire electrolytic solution in the container is electrolyzed at all times.

### SUMMARY OF THE INVENTION

In the light of the above shortcomings, it is an object of the present invention to provide an electrolyzed water generating and spraying device that allows generating electrolyzed water in a short time, and allows non-deteriorated electrolyzed water to be sprayed immediately after being generated.

In order to attain the above goal, the invention set forth in claim 1 is an electrolyzed water generating and spraying device having a container, and housed therein, an electrolytic cell for generating electrolyzed water through electrolysis of an electrolytic solution by an electrode, a spraying mechanism for manually spraying the electrolyzed water generated in the electrolytic cell, and a power supply for energizing the electrode, wherein a tank for storing the electrolytic solution and the electrolytic cell are provided independently in the container, the electrolytic cell being disposed below the tank, and the tank and the electrolytic cell communicating with each other via a communicating passage.

The invention set forth in claim 2 is the invention set forth in claim 1, wherein part of at least the electrolytic cell in the container is transparent.

The invention set forth in claim 3 is the invention set forth in claim 1 or 2, wherein the volume of the electrolytic cell is set to be smaller than the volume of the tank.

The invention set forth in claim 4 is any one of the inventions set forth in claims 1 to 3, wherein the electrode is provided in plurality, the distance between electrodes being set to be short in part.

The invention set forth in claim 5 is any one of the inventions set forth in claims 1 to 4, wherein the tank and the electrolytic cell are partitioned by a partition wall, the communicating passage being formed in the partition wall on the opposite side of an inlet of the spraying mechanism with respect to the electrode.

The invention set forth in claim 6 is the invention set forth in claim 5, wherein the communicating passage is formed along a container inner wall in the partition wall, and a protrusion is formed on part of an opening edge of the partition wall, at a portion on the side of the electrolytic cell.

The invention set forth in claim 7 is any one of the inventions set forth in claims 1 to 6, wherein a light-emitting element for illuminating the electrolytic cell upon being fed with current from the power supply is provided in the container.

In the invention set forth in claim 1, the tank that stores the electrolytic solution is provided, in the container, separately from the electrolytic cell, the latter being provided below the tank, with a communicating passage connecting the tank and the electrolytic cell. As a result, there is electrolyzed only the small volume of electrolytic solution in the electrolytic cell, whereby the generated electrolyzed water can be sprayed continuously. This allows generating electrolyzed water in a short time, and allows directly spraying non-deteriorated electrolyzed water immediately after being generated.

In the invention set forth in claim 2, part of at least the electrolytic cell in the container is transparent, and hence the electrolysis condition in the electrolytic cell can be visually checked from outside.

In the invention set forth in claim 3, the volume of the electrolytic cell is set to be smaller than the volume of the tank. Therefore, the small volume of electrolytic solution in the electrolytic cell can be electrolyzed in a short time.

In the invention set forth in claim 4, the distance between respective plural electrodes is set to be short in part. This increases, as a result, the current density between those portions of adjacent electrodes standing at a shorter distance from each other, thereby enhancing electrolysis in those portions. Thus, the amount of electrolysis gas that is generated in the electrodes increases on account of this enhanced electrolysis. This electrolysis gas generation reinforces the circulating flow of electrolyzed water in the electrolytic cell, whereby the electrolyzed water is stirred more vigorously, yielding as a result electrolyzed water having a given electrolytic concentration.

In the invention set forth in claim 5, the tank and the electrolytic cell are partitioned by a partition wall, and hence non-deteriorated electrolyze water that is electrolyzed in the electrolytic cell can be directly sectioned and sprayed by the spraying mechanism. The communicating passage that communicates the tank and the electrolytic cell is formed in the partition wall, at the opposite side of an inlet of the spraying mechanism with respect to the electrodes. As a result, there forms a circulating flow, centered around the electrodes in the electrolytic cell, that prevents backflow of electrolyzed water into the tank, via the communicating passage, and prevents intrusion electrolytic solution in the tank, so that electrolyzed water having a high electrolysis concentration can be stably sprayed by way of the spraying mechanism.

In the invention set forth in claim 6, the communicating passage is formed along a container inner wall in the partition wall, and a protrusion is formed on part of an opening edge of the partition wall, at a portion on the side of the electrolytic cell. The protrusion prevents as a result backflow, into the tank, of electrolysis gas generated through electrolysis of the electrolytic solution in the electrolytic cell, so that the electrolyzed water in the electrolytic cell becomes naturally homogenized through stirring resulting from circulation of the electrolysis gas. This simplifies the structure of the device, since no shirring means is then required.

In the invention set forth in claim 7, a light-emitting element for illuminating the electrolytic cell upon being fed with current from the power supply is provided in the container. As a result, the electrolysis condition as well as the on/off state of the electrolytic cell can be viewed from outside.

### BRIEF DESCRIPTION OF THE DRAWINGS

Fig. 1 is a front-view diagram of an electrolyzed water generating and spraying device according to the present invention;
Fig. 2 is a cutaway side-view diagram of the electrolyzed water generating and spraying device according to the present invention;
Fig. 3 is a cross-sectional diagram of Fig. 2 along line A-A;
Fig. 4 is a cross-sectional perspective-view diagram of the electrolyzed water generating and spraying device according to the present invention;
Fig. 5 is a perspective-view diagram illustrating a portion (power supply unit) in the internal structure of the electrolyzed water generating and spraying device according to the present invention;
Fig. 6 is a diagram illustrating the relationship between electrolytic current and free chlorine concentration when a partition wall is provided between a tank and an electrolytic cell;
Fig. 7 is a diagram illustrating the relationship between electrolysis time and free chlorine concentration when a partition wall is provided between a tank and an electrolytic cell;
Fig. 8 is a diagram illustrating the relationship between electrolytic current and free chlorine concentration when no partition wall is provided between a tank and an electrolytic cell;
Fig. 9 is a diagram illustrating the relationship between electrolysis time and free chlorine concentration when no partition wall is provided between a tank and an electrolytic cell; and
Figs. 10A to 10C are diagrams illustrating other electrode embodiments.

### DESCRIPTION OF THE PREFERRED EMBODIMENTS

Embodiments of the present invention are explained next with reference to accompanying drawings.

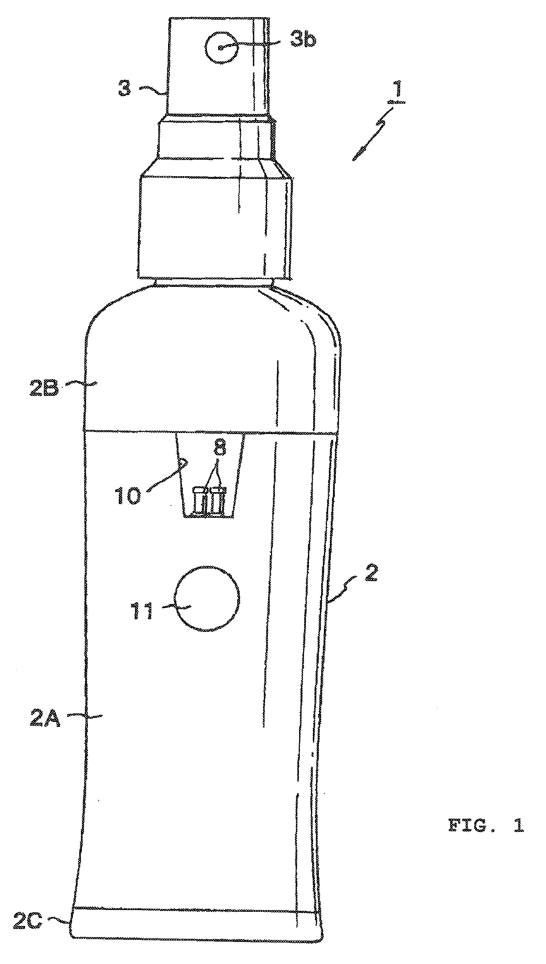

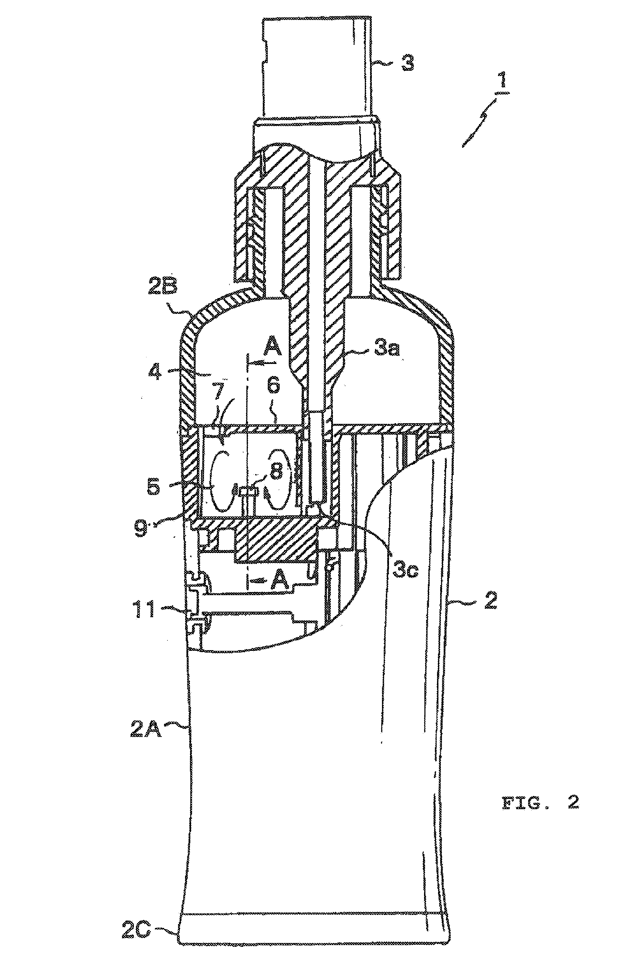

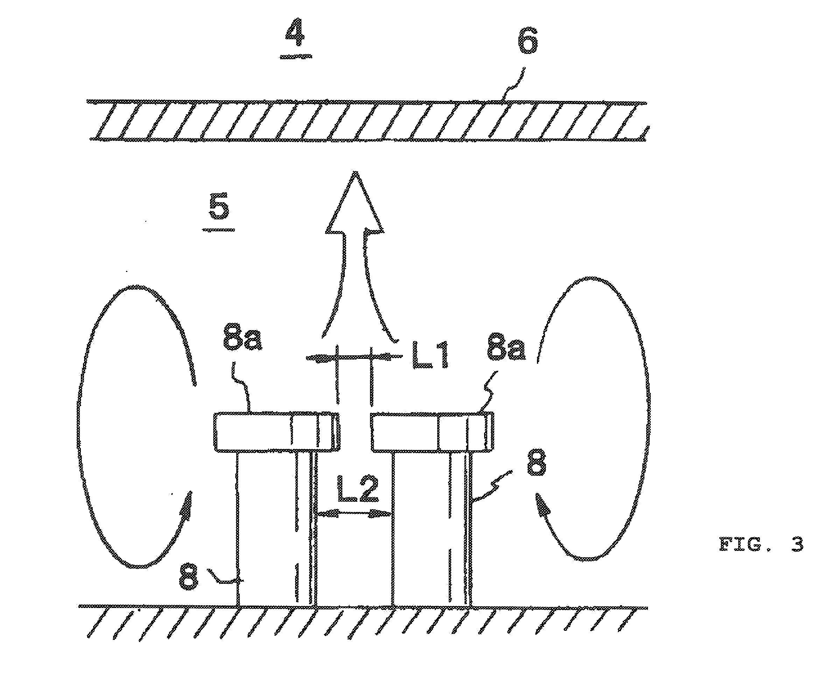

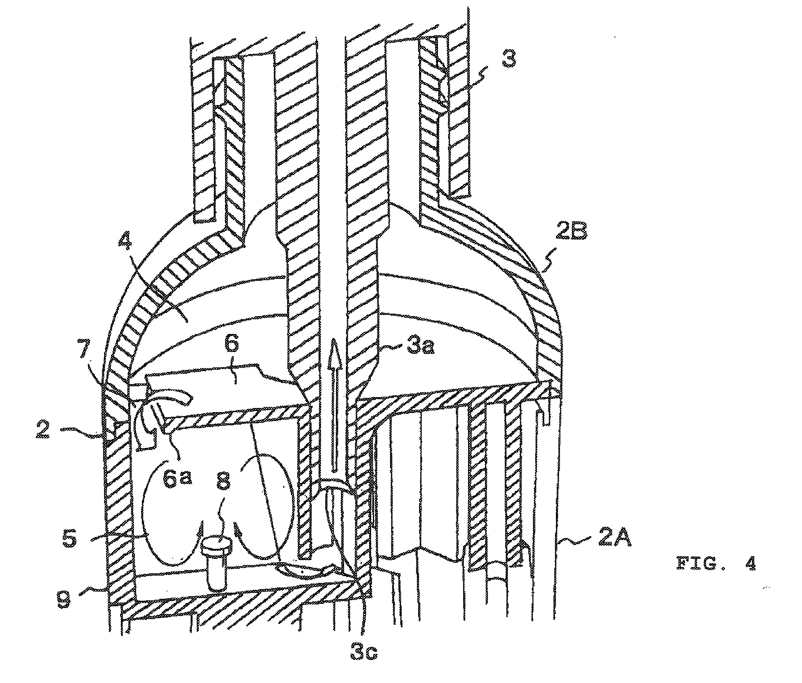

Fig. 1 is a front-view diagram of the electrolyzed water generating and spraying device according to the present invention; Fig. 2 is a cutaway side-view diagram of the electrolyzed water generating and spraying device; Fig. 3 is a cross-sectional diagram of Fig. 2 along line A-A; Fig. 4 is a cross-sectional perspective-view diagram of a characterizing portion of the electrolyzed water generating and spraying device; and Fit. 5 is a perspective-view diagram illustrating a portion (power supply unit) in the internal structure of the electrolyzed water generating and spraying device.

In the electrolyzed water generating and spraying device 1 illustrated in Figs. 2 and 2, the reference numeral 2 denotes a substantially tubular container. The container 2 comprises a tubular container main body 2A, as well as a two-stage tubular cap 2B and a circular piate-shaped cap 2C to which the upper face and the lower face of the container main body 2A are respectively fitted. A manual spray pump 3, as a spraying mechanism, is provided above the container 2.

A characterizing feature of the electrolyzed water generating and spraying device 1 according to the present invention is that a tank 4 for storing an electrolytic solution and an electrolytic cell 5 for generating electrolyzed water are provided separately in the container 2. As illustrated in Figs. 2 to 4, the tank 4 and the electrolytic cell 5 are disposed one above the other in the upper half portion of the container 2, such that a power supply unit is housed below the tank 4 and the electrolytic cell 5.

The electrolytic cell 5 is disposed directly below the tank 4. The electrolytic cell 5 and the tank 4, which are vertically partitioned by a partition wall 6, communicate with each other via a communicating passage 7 formed along the inner wall of the container 2 in the partition wall 6. As illustrated in detail in Fig. 4, a protrusion 6a is integrally formed on part of the opening edge of the partition wall 6, at a potion on the side of the electrolytic cell 5, the protrusion 6a facing into the electrolytic cell 5.

Two electrodes 8 are provided standing in the electrolytic cell 5. The volume of the electrolytic cell 5 is set to be smaller than the volume of the tank 4. A vertically-extending nozzle 3a of the manual spray pump 3 runs through the center of the tank 4, such that the lower end of the nozzle 3a opens onto the electrolytic cell 5. As illustrate in Fig. 1, moreover, a spray opening 3b is opened in the upper end of the manual spray pump 3.

In the present embodiment, flange-like large-diameter portions 8a are formed at the apexes of the electrodes 8, as illustrated in detail in Fig. 3. The distance L1 between the large-diameter portions 8a of the two electrodes 8 is set to be smaller than the distance L2 between other portions (small-diameter portions) of the electrodes 8 (L1<L2).

In the present embodiment, as described above, the communicating passage 7 is formed along the inner wall of the container 2 in the partition wall 6. As illustrated in Figs. 2 and 4, the communicating passage 7 is formed in the partition wall 6 at the opposite side of an inlet 3c of the manual spray pump 3 with respect to the electrodes 8.

In the present embodiment, a member 9 that forms part of the electrolytic cell 5 is made of a transparent resign material. Part of the member 9 makes up part of the outer surface of the container 2, in the form of a transparent window 10, as illustrated in Fig. 1. The electrodes 8 in the electrolytic cell 5 can be viewed therefore through the transparent window 10. As illustrated in Fig. 1, a circular switch 11 is disposed below the transparent window 10 on the outer surface of the container 2.

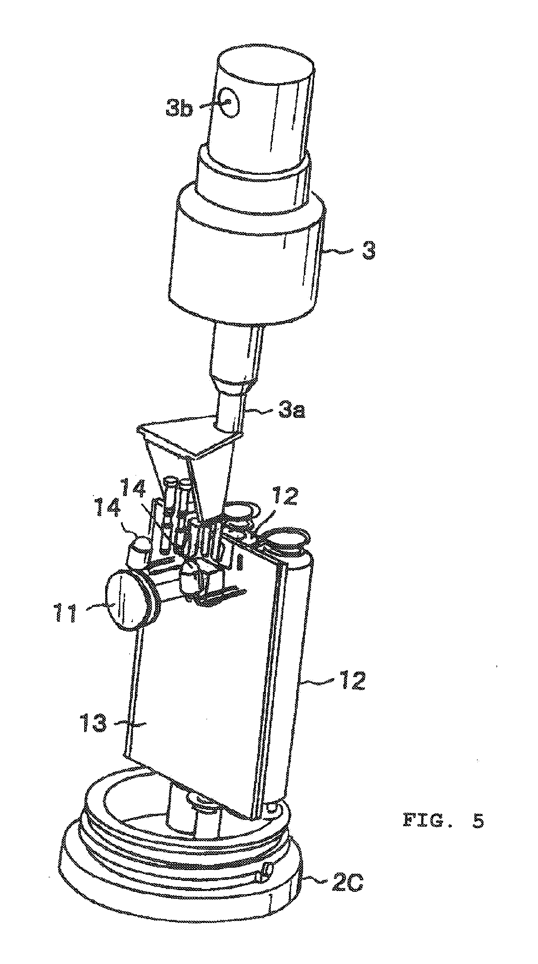

As illustrated in Fig. 5, in the container 2 there are also housed, at a portion below the electrolytic cell 5, for instance two batteries (dry-cell batteries) 12 as a power supply, a board 13 for controlling the current from the batteries 12, the above-described switch 11, and two LEDs 14, as light-emiitting elements, for illuminating the electrolytic cell 5 upon being fed with current from the batteries 12.

An electrolytic solution is held in the tank 4 and the electrolytic cell 5 of the electrolyzed water generating and spraying device 1 having the above configuration. When the user presses the switch 11, during use of the device, the electrodes 8 in the electrolytic cell 5 are energized by the batteries 12, whereupon the electrolytic solution in the electrolytic cell 5 is electrolyzed, to generate electrolyzed water in the electrolytic cell 5. When the user operates manually the manual spray pump 3, therefore, the electrolyzed water generated in the electrolytic cell 5 is directly suctioned through the inlet 3c towards the nozzle 3a of the manual spray pump 3, and is sprayed, as an atomized mist, out of the spray opening 3b. Mist-like electrolyzed water is thus sprayed and applied onto human skin.

The electrolytic cell 5 is replenished with electrolytic solution from the tank 4, via the communicating passage 7, as the amount of electrolyzed water decreases in the electrolytic cell 5 through being consumed by spraying and application. The electrolyzed water can be continuously sprayed and applied as a result.

In the electrolyzed water generating and spraying device 1 according to the present invention, the tank 4 for storing electrolytic solution is provided in the container 2 separately from the electrolytic cell 5, the latter being provided immediately below the tank 4, such that the tank 4 and the electrolytic cell 5 communicate normally with each other via the communicating passage 7. As a result, there is electrolyzed only the small volume of electrolytic solution in the electrolytic cell 5, whereby the generated electrolyzed water can be sprayed continuously. This allows generating electrolyzed water in a short time, and allows directly spraying non-deteriorated electrolyzed water, immediately after being generated, through the manual spray pump 3. In the present embodiment, the volume of the electrolytic cell 5 is set to be smaller than the volume of the tank 4, thanks to which the small volume of electrolytic solution in the electrolytic cell 5 can be electrolyzed in a short time. The degree of electrolysis can be adjusted by adjusting the duration of electrolysis before spraying.

In the present embodiment, the member 9 that forms part of the electrolytic cell 5 is made of a transparent resin material. As illustrated in Fig. 1, part of the member 9 is exposed at the outer surface of the container 2, in the form of the transparent window 10. This allows therefore viewing the electrodes in the electrolytic cell 5, through the transparent window 10, so that the electrodes 8 in the electrolytic cell 5 as well as the electrolysis condition in the electrolytic cell 5 can be visually checked from outside.

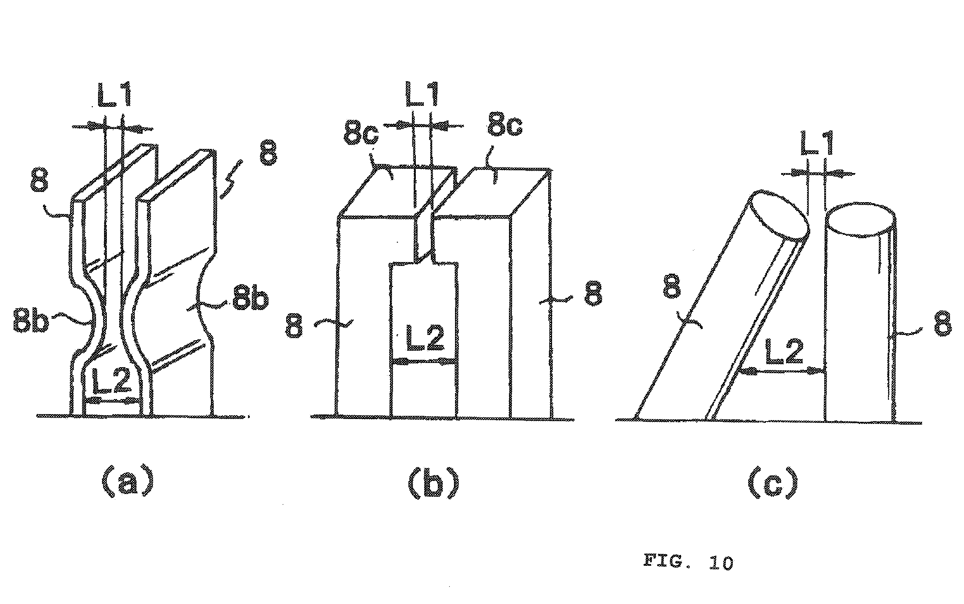

In the present embodiment, moreover, the flange-like large-diameter portions 8a are formed at the apexes of the electrodes 8, as illustrated in Fig. 3, such that a distance L1 between the large-diameter portions 8a of the two electrodes 8 is set to be smaller than the distance L2 between other portions (small-diameter portions) of the electrodes 8 (L1<L2). This increases, as a result, current density between the large-diameter portions 8a, thereby enhancing electrolysis in that portion. Thus, the amount of electrolysis gas that is generated in the electrodes 8 increases on account of this enhanced electrolysis. This electrolysis gas generation reinforces the circulating flow of electrolyzed water in the electrolytic cell 5, whereby the electrolyzed water is stirred more vigorously, yielding as a result electrolyzed water having a given electrolytic concentration. Although in the present embodiment the flange-like large-diameter portions 8a are formed at the apexes of the electrodes 8, so that the distance L1 between the large-diameter portions 8a of the two electrodes 8 is set to be smaller than the distance L2 between other portions (small-diameter portions) of the electrodes 8 (L1<L2), the distance between the adjacent electrodes 8 need only be set to be short in part in order to elicit the above effect. For instance, mutually-facing arcuate projections 8b may be formed at an intermediate portion, in the height direction, of the electrodes 8, as illustrated in Fig. 10A, so that the distance L1 between the projections 8b is set to be shorter than the distance L2 between other portions of the electrodes 8. Likewise, quadrangular prism-shaped projections 8c may be formed at each apex of the electrodes 8, as illustrated in Fig. 10B, so that the distance L1 between the projections 8c is set to be shorter than the distance L2 between other portions of the electrodes 8. Alternatively, one of the solid cylindrical electrode 8 may slat as illustrated in Fig, 10C, so that the distance L1 between the apexes of the two electrodes is shorter than the distance L2 between other portions of the electrodes 8.

In the present embodiment, the communicating passage 7 is formed, along the inner wall of the container 2, in the partition wall 6 that partitions the tank 4 and the electrolytic cell. 5, with the protrusion 6a being formed on part of the opening edge of the partition wall 6, at a portion on the side of the electrolytic cell 5. The protrusion 6a prevents backflow, into the tank 4, of electrolysis gas generated through electrolysis of the electrolytic solution in the electrolytic cell 5, so that the electrolyzed water in the electrolytic cell 5 becomes naturally homogenized through stirring resulting from circulation of the electrolysis gas. This simplifies the structure of the device, since no stirring means is then required.

In the present embodiment, moreover, the LEDs 14 for illuminating the electrolytic cell 5 upon being fed with current by the batteries 12 are provided in the container 2. The electrolysis condition in the electrolytic cell 5, as well as the on/off state of the switch 11, can thus be viewed from outside.

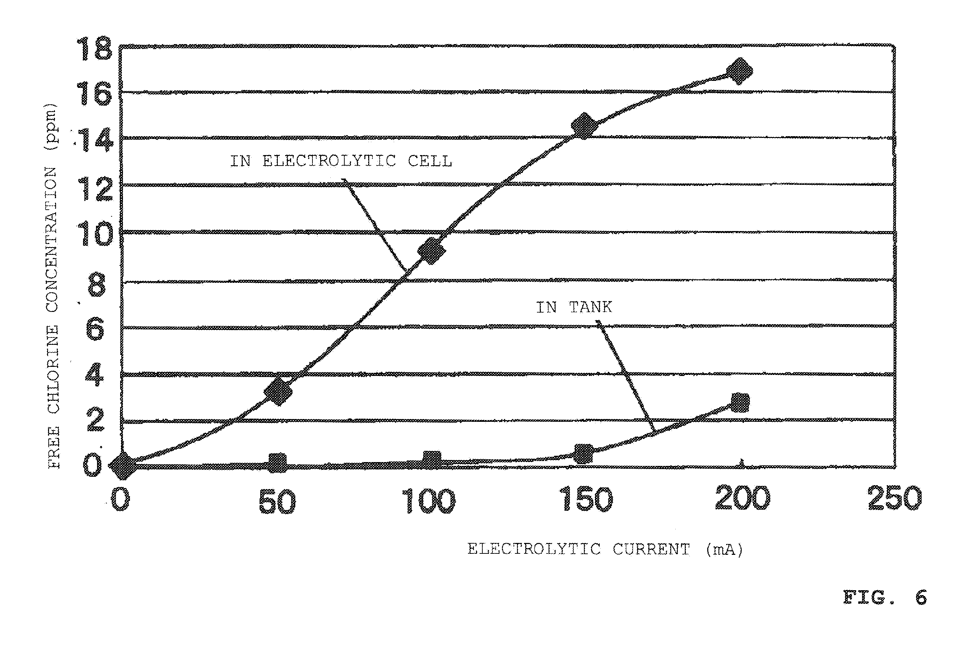

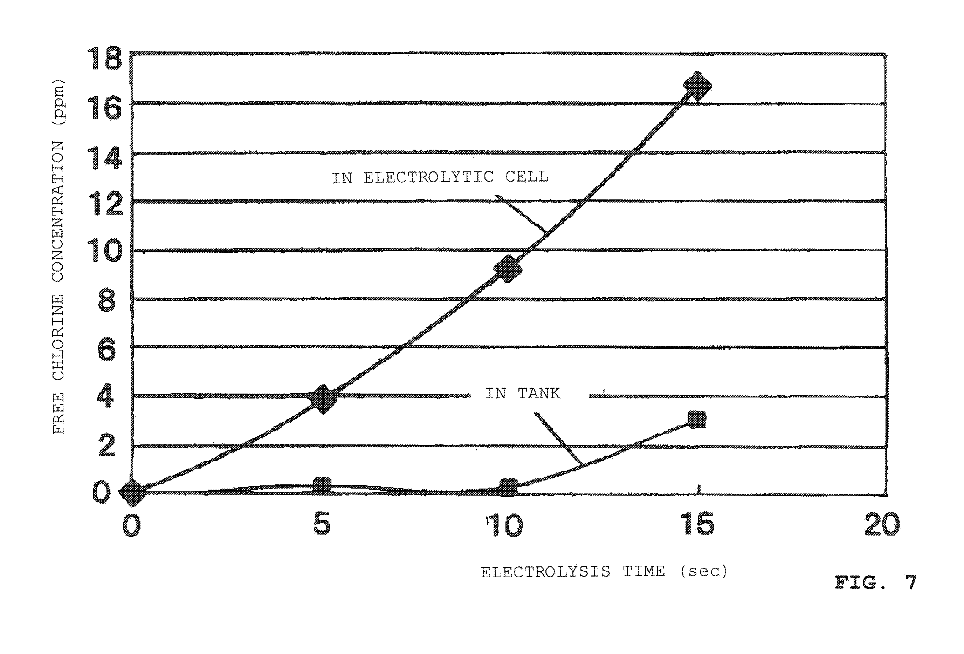

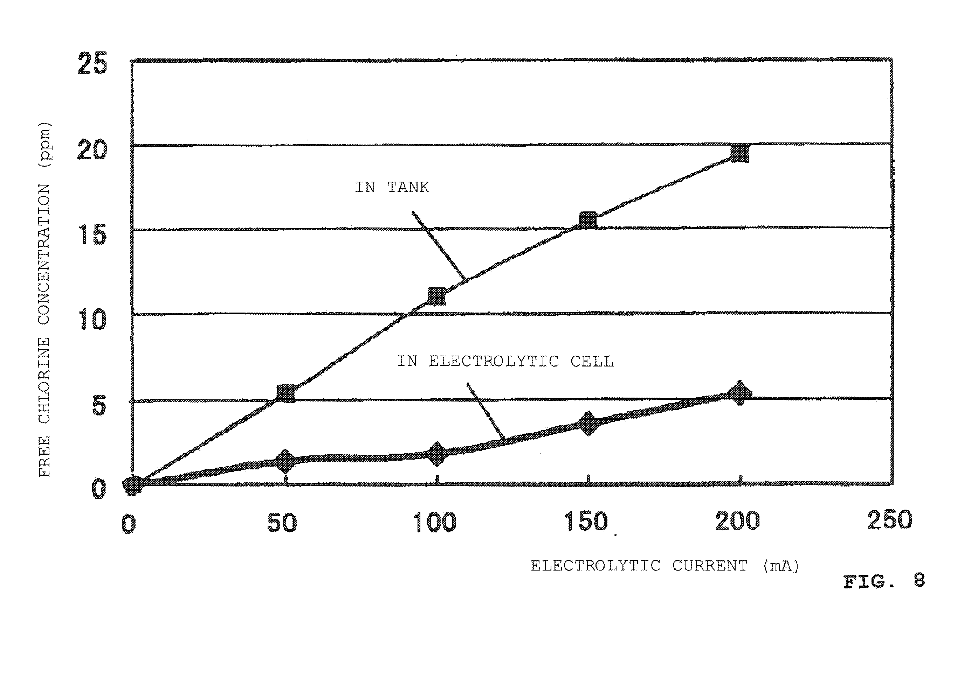

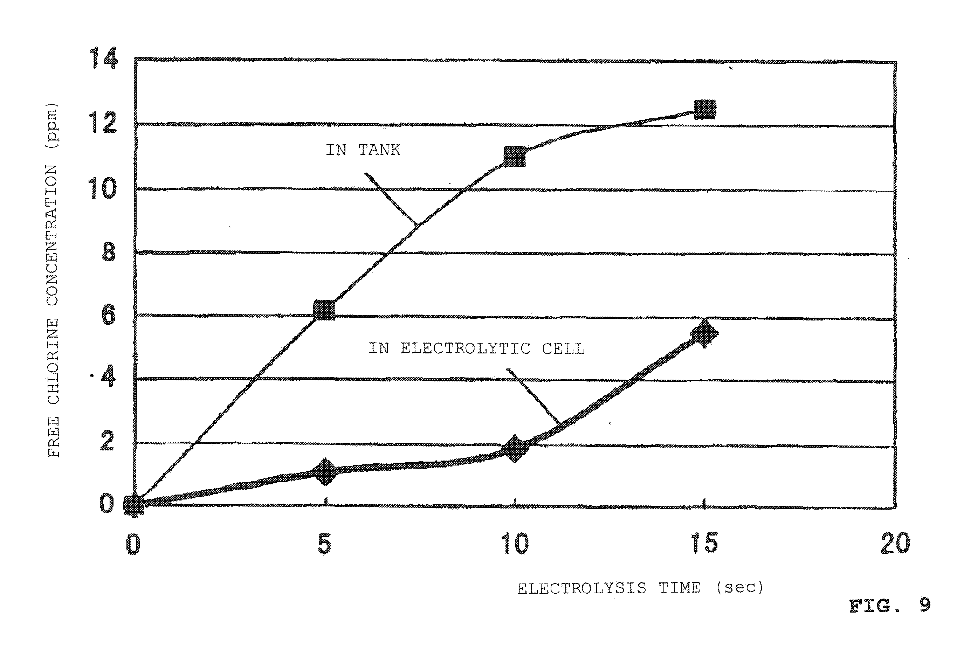

Figs. 6 to 9 illustrated the results of experiments performed on the electrolyzed water generating and spraying device 1 according to the present invention. Fig. 6 is a diagram illustrating the relationship between electrolytic current and free chlorine concentration when a partition wall is provided between the tank and the electrolytic cell; Fig. 7 is a diagram illustrating the relationship between electrolysis time and free chlorine concentration when a partition wall is provided between the tank and the electrolytic cell; Fig. 8 is a diagram illustrating the relationship between electrolytic current and free chlorine concentration when no partition wall is provided between the tank and the electrolytic cell; and Fig. 9 is a diagram illustrating the relationship between electrolysis time and free chlorine concentration when no partition wall is provided between the tank and the electrolytic cell.

In the experiments there was used a device having a tank capacity of 5 ml, an electrolytic cell capacity of 3 ml, and there was measured the free chlorine concentration in the electrolyzed water generated as a result of 10-second electrolysis by varying the electrolytic current (mA) in an electrolytic solution comprising 2500 ppm of NaCl, for a case in which a partition wall was provided between the tank and the electrolytic cell, and a case in which no such partition wall was provided. The results are illustrated in Figs. 6 and 8.

The specific gravity of the free chlorine generated through electrolysis in the electrolytic cell is smaller than the specific gravity of the NaCl electrolytic solution, and hence the free chlorine rises up at once. When the partition wall is provided, therefore, the free chlorine in the electrolytic cell does not diffuse into the tank but remains in the electrolytic cell. As a result, while the concentration of free chlorine in the electrolytic cell where electrolyzed water is generated rises as the electrolytic current increases, virtually no free chlorine concentration is measured in the tank that stores the NaCl electrolytic solution, as illustrated in Fig. 6. A small free chlorine concentration in the tank becomes measurable for an electrolytic current in excess of 150 mA. When no partition wall is provided, by contrast, the free chlorine cannot be confined to the electrolytic cell and diffuses into the tank. As a result, the free chlorine concentration in the tank that stores the NaCl electrolytic solution rises in substantially direct proportion to the increase in electrolytic current, whereas the free chlorine concentration increases only slightly in the electronic component where the electrolyzed water is generated, as illustrated in Fig. 8, so that the above effect is reduced by half.

Figs. 7 and 9 illustrate the change over time of the free chlorine concentration when a NaCl solution is electrolyzed with the electrolytic current set to a constant 100 mA. When the partition wall is provided, the free chlorine in the electrolytic cell increases hyperbolically over time (see), whereas virtually no free chlorine concentration is measured in the tank, in which a slight increase in free chlorine concentration is measured only after 10 seconds. When no partition wall is provided, by contrast, the free chlorine concentration rises gradually over time in the electrolytic cell, where the electrolyzed water is generated, while the free chlorine concentration increases hyperbolically over time in the tank that stores the NaCl electrolytic solution, as illustrated in Fig. 9.

The above results indicate that the partition wall 6 is indispensable for maintaining a high free chlorine concentration in the electrolyzed water generated in the electrolytic cell 5. When the communicating passage 7 that communicates normally the tank 4 and the electrolytic cell 5 is formed in the partition wall 6, on the opposite side of the inlet 3c of the manual spray pump 3, flanking the electrodes 8, as is the case in the present embodiment, there forms as a result a circulating flow centered around the electrodes 8 in the electrolytic cell 5, such as the flow denoted by the arrows in Figs. 2 to 4. This circulating flow prevents backflow, into the tank 4, of electrolyzed water having a high free chlorine concentration via the communicating passage 7, and prevents intrusion of NaCl solution, having a low free chlorine concentration, in the tank 4, so that electrolyzed water having a high free chlorine concentration can be stably sprayed by way of the manual spray pump 3.

In summary, an embodiment of the invention provides an electrolyzed water generating and spraying device that allows generating electrolyzed water in a short time, and allows spraying non-deteriorated electrolyzed water immediately after being generated. An electrolyzed water generating and spraying device has a container, and housed therein, an electrolytic cell for generating electrolyzed water through electrolysis of an electrolytic solution by an electrode, a manual spray pump (spraying mechanism) for manually spraying the electrolyzed water generated in the electrolytic cell, and a power supply (battery) for energizing the electrode. A tank for storing the electrolytic solution and the electrolytic cell are provided independently in the container, such that the electrolytic cell is disposed below the tank, and the tank and the electrolytic cell communicate with each other via a communicating passage. Part of at least the electrolytic cell in the container is transparent. Also, the volume of the electrolytic cell is set to be smaller than the volume of the tank.

## Claims

1. An electrolyzed water generating and spraying device comprising a container, and housed therein, an electrolytic cell for generating electrolyzed water through electrolysis of an electrolytic solution by an electrode, a spraying mechanism for manually spraying the electrolyzed water generated in the electrolytic cell, and a power supply for energizing said electrode,
wherein a tank for storing said electrolytic solution and said electrolytic cell are provided independently in said container, said electrolytic cell being disposed below said tank, and said tank and said electrolytic cell communicating with each other via a communicating passage.

2. The electrolyzed water generating and spraying device according to claim 1, wherein part of at least said electrolytic cell in said container is transparent.

3. The electrolyzed water generating and spraying device according to claim 1 or 2, wherein the volume of said electrolytic cell is set to be smaller than the volume of said tank.

4. The electrolyzed water generating and spraying device according to any one of claims 1 to 3, wherein said electrode is provided in plurality, the distance between electrodes being set to be short in part.

5. The electrolyzed water generating and spraying device according to any one of claims 1 to 4, wherein said tank and said electrolytic cell are partitioned by a partition wall, said communicating passage being formed in said partition wall on the opposite side of an inlet of said spraying mechanism with respect to said electrode.

6. The electrolyzed water generating and spraying device according to claim 5, wherein said communicating passage is formed along a container inner wall in said partition wall, and a protrusion is formed on part of an opening edge of said partition wall, at a portion on the side of said electrolytic cell.

7. The electrolyzed water generating and spraying device according to any one of claims 1 to 6, wherein a light-emitting element for sail electrolytic cell upon being fed with current from said power supply is provided in said container.
